# EUROPÄISCHE PATENTSCHRIFT

(11) **EP 0 452 790 B1**
(45) Veröffentlichungstag und Bekanntmachung des Hinweises auf die Patenterteilung: **22.06.1994**
(21) Anmeldenummer: 91105661.2
(22) Anmeldetag: 10.04.1991
(51) Int. Cl.: B01L 11/00, A47L 15/00, A47L 15/50

(54) **Verfahren zum Reinigen und Trocknen von in einem Einsatzwagen einer Labor-Geschirrspülmaschine abgelegten Atemschläuchen und Einsatzwagen zur Durchführung des Verfahrens**
Process for cleaning and drying of respiration tubes deposited into an insertion rack of a laboratory-dishwasher and insertion rack for carrying out the process
Procédé pour nettoyer et sécher des tubes de respiration déposés dans un chariot d'insertion de machine à laver la vaisselle de laboratoire et chariot d'insertion pour la réalisation du procédé

(30) Priorität: 14.04.1990 DE 4012138
(43) Veröffentlichungstag der Anmeldung: 23.10.1991
(73) Patentinhaber: Miele & Cie. GmbH & Co., D-33332 Gütersloh (DE)
(72) Erfinder: Meise, Bruno, W-4800 Bielefeld 18 (DE); Michels, Winfried, Dr., W-4830 Gütersloh 1 (DE); Risse, Gerhard, W-4800 Bielefeld 1 (DE)

(56) Entgegenhaltungen:
- DE-A- 2 226 894
- DE-A- 3 143 005
- DE-A- 3 443 912
- DE-A- 3 710 349

## Beschreibung

### Verfahren zum Reinigen und Trocknen von in einem Einsatzwagen einer Labor-Geschirrspülmaschine abgelegten Atemschläuchen und Einsatzwagen zur Durchführung des Verfahrens

Die Erfindung betrifft ein Verfahren zum Reinigen und Trocknen von Atemschläuchen in einer programmgesteuerten Labor-Geschirrspülmaschine, wobei die Atemschläuche auf einem als Einsatzwagen ausgebildeten Traggestell mit Spülwasser- und Trocknungsluft-Rohranschlüssen sowie zugeordneten Düsensätzen in ganzer Länge steigend spiralförmig oder wendelförmig abgelegt sind und wobei die Reinigungs- oder Spülflüssigkeit sowie die Trocknungsluft in den jeweiligen Programmschritten den Atemschläuchen endseitig zugeführt wird. Ferner betrifft die Erfindung einen Einsatzwagen zur Durchführung des Verfahrens.

Zur Aufbereitung von Atemschläuchen in programmgesteuerten Labor-Geschirrspülmaschinen ist aus der DE-A- 37 10 349 ein Verfahren zum Reinigen, Spülen, Desinfizieren und Trocknen bekannt, nach welchem die in einem Einsatzwagen der Geschirrspülmaschine spiral- oder wendelförmig abgelegten flexiblen Atemschläuche von oben nach unten von einer Reinigungs- und Spülflotte sowie anschließend von Trocknungsluft zur Innenreinigung durchströmt werden. Die Außenreinigung der Atemschläuche erfolgt über Sprüharme oder Sprühdüsen, welche auf die zu reinigenden Schlauchhohlkörper gerichtet sind.

Bei dem bekannten Verfahren hat es sich als nachteilig herausgestellt, daß die in Fließrichtung der Spülflotte gesehenen hinteren Bereiche der inneren Schlauchfalten der faltenbalgähnlich gestalteten Atemschläuche nur mit einer übermäßig hohen Wasser- und Trocknungsluftversorgung optimal gereinigt sowie getrocknet werden können, da diese Schlauchfaltenbereiche von dem vorbeigeführten Medium nicht unmittelbar kontaktiert werden. Darüber hinaus kann es trotzdem noch vorkommen, daß bei ungünstiger Schlauchablage im Einsatzwagen bzw. bei nicht ordnungsgemäß auf die Spritzdüsen ausgerichteten Schlauchenden die Atemschläuche unzureichend mit ggfs. auch nach dem Trocknen zurückbleibenden Wassernestern in den Schlauchfalten gereinigt werden, so daß eine zeitraubende Nachbehandlung der Atemschläuche erforderlich wird.

Aus der DE-A- 31 43 005 ist ferner ein Verfahren zur Atemschlauch-Durchspülung bekannt, bei welchem die Schläuche auf den Außenradius einer rotierenden Trommel abgelegt sind und jeweils mit einem Ende vor einer das Spülwasser bzw. die Trocknungsluft führenden Injektordüse plaziert sind. Auch bei diesem Verfahren werden die Innenräume der aufgewickelten Atemschläuche vom Spülwasser unzureichend durchströmt, wobei sich die Trommel während des Reinigungsvorgangs dreht. Das Spülwasser wird dabei unter Mitnahme der Anschmutzungen aus den Atemschläuchen ausgeschwemmt. Während des Trocknungsvorgangs wird die Trommel mit den Schläuchen weitergedreht.

Ein solches Verfahren setzt jedoch einen technisch sehr aufwendigen Spezialeinsatz für die Aufnahme der aufzubereitenden Atemschläuche voraus, in dem für weiteres zu reinigendes Anästhesie-Zubehör, wie Atemkalkbehälter, Atembeutel und dgl. keine geeignete Stellfläche mehr abgetrennt werden kann. Ebenfalls ist bei diesem bekannten Verfahren bei nicht ordnungsgemäß plazierten Atemschläuchen die optimale Durchflutung der Schläuche nicht sichergestellt.

Der Erfindung liegt die Aufgabe zugrunde, ein Verfahren zur hygienischen Aufbereitung bzw. zum Reinigen, Desinfizieren und Trocknen von Atemschläuchen zu schaffen, welches mit vertretbarem Aufwand hinsichtlich der Ausbildung des Einsatzwagens sowie hinsichtlich Zeit- und Energieverbrauch eine optimale Durchflutung der Schlauchhohlkörper unabhängig von deren Länge, Durchmesser oder Schlauchfaltenausbildung sicherstellt.

Diese Aufgabe wird erfindungsgemäß gelöst durch die Merkmale des Anspruchs 1.

Ein entsprechender Einsatzwagen zur Durchführung des Verfahrens sowie vorteilhafte Weiterbildungen davon sind den weiteren Ansprüchen zu entnehmen.

Die Erfindung gewährleistet eine 100 %ige Innenspülung und somit auch Innenreinigung der Atemschläuche dadurch, daß diese in ihrer Länge steigend kreisförmig oder spiralförmig auf einem Traggestell des Einsatzwagens abgelegt werden und von unten nach oben von einer Reinigungs- bzw. Spülflottensäule durchströmt werden. Diese Spülflottensäule wird aufgebaut, indem das untere Schlauchende an eine Düsenvorrichtung adaptiert wird, die einen größeren Flottenauslauf an dieser Stelle verhindert. Ebenso wie der Aufbau der Reinigungs- bzw. Spülflottensäule wird auch im Programmabschnitt "Trocknen" eine Trocknungsluftsäule aufgebaut, welche dafür sorgt, daß auch die Trocknung der Atemschläuche optimal ohne Zurückbleiben von Wassernestern durchgeführt wird. Durch die spiralförmige bzw. kreis- oder wendelförmige Ablage der Atemschläuche im Zentrum des Einsatzwagens werden Stellflächen für weiteres Anästhesiezubehör, wie Atemkalkbehälter, Atembeutel und dgl. geschaffen, wobei für auch dieses Zubehör entsprechende Einspritzvorrichtungen mit speziellen Düsen im Wagen vorgesehen werden können. Mit dem Einsatzwagen gemäß der Erfindung können somit auch verschiedene Instrumente für den Krankenhaus- bzw. Laborbedarf praxisgerecht aufbereitet werden.

In der nachfolgenden Beschreibung ist das erfindungsgemäße Verfahren näher erläutert und ein Einsatzwagen einer Labor-Geschirrspülmaschine zur Durchführung des Verfahrens in einem Ausführungsbeispiel zeichnerisch dargestellt. Es zeigt:
- Figur 1: einen Einsatzwagen einer Labor-Geschirrspülmaschine zur Aufbereitung von Atemschläuchen in der Vorderansicht,
- Figur 2: den Einsatzwagen nach Figur 1 in perspektivischer Darstellung mit mehreren in einem Traggestell zur Aufbereitung abgelegten Atemschläuchen.

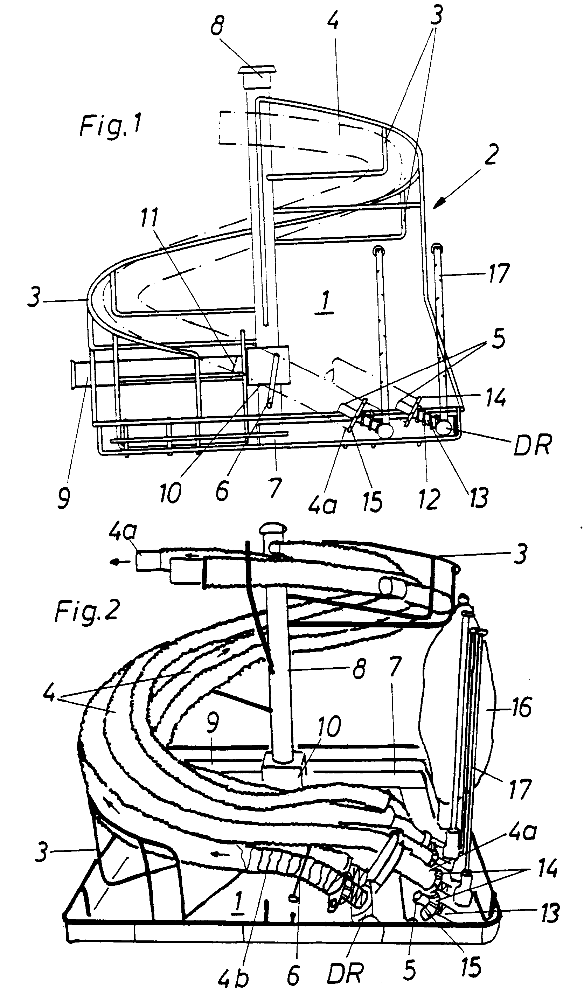

Ein in den Figuren 1 und 2 dargestellter Einsatzwagen (1) für eine nicht gezeigte Labor-Geschirrspülmaschine zum Reinigen, Desinfizieren und Trocknen von Anästhesiezubehör weist ein Traggestell (2) mit Schlauchablagen (3) für zu reinigende Atemschläuche (4) auf.

Die Atemschläuche (4) sind vor der hygienischen Aufbereitung ihrer Länge nach steigend kreisförmig oder wendelförmig auf dem Traggestell (2) abzulegen. Dafür sind die Schlauchablagen (3), beginnend im oberen Wagenteil, ebenfalls in spiral- oder wendelförmiger Anordnung abwärsts gerichtet ausgebildet. Die untere Schlauchablage (3) endet vor separaten, jeweils zu Düsengruppenreihen (DR) zusammengefaßten Spülwasser- und Trocknungsluftdüsen (5), an welche die abgelegten Atemschläuche undicht (s. auch Figur 2) ankuppelbar sind. Das undichte Ankuppeln der Atemschläuche (4) sichert das Abfließen von Spülwasser bzw. Reinigungswasser nach dem Reinigungs/Spülgang aus den Schläuchen.

Zumindest die untere Schlauchablage (3) des Traggestells (2) ist zur Aufnahme mehrer Atemschläuche (4) in nebeneinander und/oder übereinander liegender Folge ausgebildet. Für jeden abgelegten Atemschlauch (4) ist im Bereich vor den Spülwasser- und Trocknungsluftdüsen ein Schlauchhalter (6) zugeordnet. Die Atemschläuche (4) sind mit ihren unteren Atemschlauchenden (4a) auf die Spülwasser- und Trocknungsluftdüsen (5) gesteckt. Die Düsengruppenreihen (DR) sind vorzugsweise hintereinander in einer Ebene vor den Schlauchhaltern (6) angeordnet und an ein gemeinsames Zuleitungsrohrsystem (7) des Einsatzwagens (1) angeschlossen, wobei das gemeinsame Zuleitungsrohrsystem (7) sich in separate Leitungswege (8, 9) für das von der Geschirrspülmaschine gelieferte Spülwasser bzw. für die von der Geschirrspülmaschine gelieferte Trocknungluft verzweigt. Nicht dargestellte Sprüharme oder Sprühdüsen und Luftdüsen am oder im Einsatzwagen (1) sorgen für eine ordnungsgemäße Außenreinigung der Atemschläuche (4).

Die vorerwähnten Schlauchablagen (3) für die Atemschläuche (4) sind um einen zentralen Rohrstutzen (10) des Traggestells (2) herum angeordnet. Der Rohrstutzen (10) wird von oben über den mit Reinigungs- oder Spülwasser versorgten Leitungsweg (8) gespeist. An den Rohrstutzen (10) ist ebenfalls der andere Leitungsweg (9) für die Zuführung der Trocknungsluft angeschlossen, in welchem Leitungsweg ein Ventil (11) vorgesehen ist. Das Ventil (11) verhindert, daß über den Leitungsweg (8) zugeführtes Reinigungs- oder Spülwasser in den Leitungsweg (9) für die Trockungsluft gelangt. Sowohl der Leitungsweg (8) als auch der Leitungsweg (9) mündet in das gemeinsame Zuleitungsrohrsystem (7), welches die Düsengruppenreihen (DR) speist.

Für die Zufuhr der Reinigungs- und Spülflüssigkeit sowie für die Zufuhr der Trockungsluft zu den Atemschläuchen (4) ist immer eine gemeinsame Düse (5) innerhalb einer Düsengruppenreihe (DR) vorgesehen, wobei jede einem Atemschlauchende (4a) zugeordnete Düse (5) mit einer in Düsenlängsrichtung gegen Federdruck (Feder 12) ausweichbaren, konzentrisch auf dem Düsenschaft (13) angeordneten Verschlußplatte (14) für einen Atemschlauch (4) versehen ist. Die Verschlußplatte (14) weist Öffnungen (15) oder Ausnehmungen in Form von Bohrungen, Rillen oder dgl. auf, welche den undichten unteren Atemschlauchabschluß bewirken. Aus diesem undichten Abschluß kann nach dem Reinigungs- oder Spülgang das noch im Atemschlauch (4) befindliche Restwasser ungehindert abfließen.

Durch die zentrale Anordnung der Schlauchablagen (3) im Einsatzwagen (1) wird in vorteilhafter Weise genügend Stellfläche für weiteres Spülgut (16), wie Atembälge, Atemkalkgeräte, Intubationshohlkörper oder dgl. geschaffen. Diesem Spülgut sind spezielle Reinigungs- und Trocknungsluftdüsen (17) in vertikaler oder abgewinkelter Form, welche das zu reinigende Spülgut (16) tragen, zur Innenreinigung zugeordnet, wobei die Düsen (17) gleichermaßen wie die Spülwasser- und Trocknungsluftdüsen (S) für die Atemschläuche (4) vom Zuleitungsrohrsystem (7) des Einsatzwagens (1) gespeist sind.

Zur hygienischen Aufbereitung werden die in ihrer Länge in die Schlauchablagen (3) steigend spiralförmig oder wendelförmig gelegten Atemschläuche in den wasserführenden Programmabschnitte "Reinigen/Spülen" eines in bekannter Weise ausgewählten Reinigungs- und Desinfektionsprogramms mit anschliessender Trocknung von einer Reinigungs- oder Spülflottensäule durchströmt. Im Programmabschnitt "Trocknen" wird eine entsprechende Trocknungsluftsäule in den Atemschläuchen (4) aufgebaut.

Diese Spülflottensäule wird aufgebaut, indem dem unteren Atemschlauchende (4a) das Spülwasser oder Reinigungswasser über die Düsen (5) zugeführt wird. Dabei tritt durch die Undichtigkeiten weniger Wasser aus als zugeführt wird. Die Spülflottensäule füllt den Atemschlauch (4) vollständig, gelangt bis zum oberen Atemschlauchende (4a) und tritt dort wieder aus. Alle Schlauchfalten (4b) innerhalb des Atemschlauchs (4) werden dabei optimal berührt und gereinigt. Die Düsen (5) der Düsengruppenreihen (DR) sind vorteilhaft so ausgebildet, daß die Kontaktflächen mit den Atemschläuchen zur Gewährleistung des geforderten Reinigungs- und Desinfektionsergebnisses möglichst klein gehalten sind. Die Düse (5) selbst ist mit einem Düsendurchmesser größer als 6 mm ausgebildet, so daß zur Reinigung des Atemschlauches (4) mit hinreichendem Volumenstrom gespült werden kann. Da bei handelsüblichen Atemschläuchen (4) die Durchmesser der Atemschlauchenden (4a) zwischen 6 mm und 30 mm variieren, ist die undichte Verschlußplatte (14) im Durchmesser größer als 30 mm ausgebildet. Die Undichtheit der Verschlußplatte (14) sorgt in den Flottenabpumpphasen, daß das Spülwasser verhältnismäßig schnell aus den Atemschläuchen auslaufen kann. Jedenfalls ist die durch einen Atemschlauch (4) strömende Wasser- oder Luftmenge weitaus größer als die an der Undichtstelle austretende Wasser- oder Luftmenge.

Durch die Erfindung wird eine 100 %ige Durchflutung der Hohlkörper-Instrumente bewirkt, wobei Schlauchdurchmesser, Atemschlauchfalten (4b), Schlauchqualität sowie die Atemschlauchanschlußstücke unberücksichtigt bleiben können. Neben den Atemschläuchen (4) kann weiteres Anästhesie-Zubehör in den Einsatzwagen praxisgerecht aufbereitet werden, wobei ggf. auch bei den weiteren Hohlkörper-Instrumenten, die nicht Atemschläuche (4) sind, ebenfalls das erfindungsgemäße Verfahren zur Bildung von Reinigungs-, Spülflotten- und Trocknungsluftsäulen verwendet werden kann.

## Patentansprüche

1. Verfahren zum Reinigen und Trocknen von Atemschläuchen (4) in einer programmgesteuerten Labor-Geschirrspülmaschine, wobei die Atemschläuche (4) auf einem als Einsatzwagen (1) ausgebildeten Traggestell (2) mit Spülwasser- und Trocknungsluft- Rohranschlüssen (7,8,9) sowie zugeordneten Düsensätzen (5,DR) in ganzer Länge steigend spiralförmig oder wendelförmig abgelegt sind und wobei die Reinigungs- oder Spülflüssigkeit sowie die Trocknungsluft in den jeweiligen Programmschritten den Atemschläuchen (4) endseitig zugeführt wird,
dadurch gekennzeichnet,
daß die Atemschläuche (4) von unten nach oben im Programmabschnitt "Reinigen/Spülen" von einer Reinigungs- oder Spülflottensäule und im Programmabschnitt "Trocknen" von einer Luftsäule durchströmt werden.

2. Einsatzwagen zur Durchführung des Verfahrens nach Anspruch 1,
dadurch gekennzeichnet,
daß das Traggestell (2) für die Atemschläuche (4) ein oder mehrere Schlauchablagen (3) in steigend spiralförmiger oder wendelförmiger Anordnung besitzt, daß die eine oder mehreren Schlauchablagen (3) unter vor separaten jeweils zu Düsengruppenreihen (DR) zusammengefaßten Spülwasser- und Trocknungsluftdüsen (5) enden, an welche die Atemschläuche (4) zum Entleeren von Spülflüssigkeit undicht ankuppelbar sind.

3. Einsatzwagen nach Anspruch 2,
dadurch gekennzeichnet,
daß zumindest die untere Schlauchablage (3) des Traggestells (2) zur Aufnahme mehrerer Atemschläuche (4) in nebeneinander und/oder übereinander liegender Folge ausgebildet ist, und daß jedem Atemschlauchende (4a) im Bereich vor den Spülwasser- und Trocknungsluftdüsen (5) ein Schlauchhalter (6) zugeordnet ist.

4. Einsatzwagen nach einem der Ansprüche 2 oder 3,
dadurch gekennzeichnet,
daß für die Zufuhr der Reinigungs- und Spülflüssigkeit sowie für die Zufuhr der Trocknungsluft zu den Atemschläuchen (4) gemeinsame Düsen (5) vorgesehen sind.

5. Einsatzwagen nach Anspruch 3 und 4,
dadurch gekennzeichnet,
daß die Düsengruppenreihen (DR) hintereinander vorzugsweise in einer Ebene vor den Schlauchhaltern (6) angeordnet und an ein gemeinsames Zuleitungsrohrsystem (7) des Einsatzwagens (1) mit separaten Leitungswegen (8, 9) für die Trocknungsluft und das Spülwasser angeschlossen sind.

6. Einsatzwagen nach einem oder mehreren der Ansprüche 2 bis 4,
dadurch gekennzeichnet,
daß die Schlauchablagen (6) für die Atemschläuche (4) um einen zentralen von oben über den einen Leitungsweg (8) mit Reinigungs- oder Spülwasser versorgten Rohrstutzen (10) des Traggestells (2) herum angeordnet sind, von welchem der andere ventilgesteuerte Leitungweg für die Zuführung der Trocknungsluft abzweigt.

7. Einsatzwagen nach einem oder mehreren der Ansprüche 2 bis 6,
dadurch gekennzeichnet,
daß jede einem Atemschlauch (4) zugeordnete Düse (5) mit einer in Düsenlängsrichtung gegen Federdruck (Feder 12) ausweichbaren, konzentrisch auf dem Düsenschaft (13) angeordneten Verschlußplatte (14) für ein Atemschlauchende (4a) versehen ist.

8. Einsatzwagen nach einem oder mehreren der Ansprüche 2 bis 7,
dadurch gekennzeichnet,
daß die Verschlußplatte (14) mit Öffnungen (15) oder Ausnehmungen in Form von Bohrungen, Rillen oder dgl. zur Realisierung des undichten unteren Atemschlauchabschlusses versehen ist.

9. Einsatzwagen nach einem oder mehreren der Ansprüche 2 bis 8,
dadurch gekennzeichnet,
daß der übrige Raum des Einsatzwagens (1) zur Aufnahme von zusätzlichem Spülgut, wie Atembälge, Atemkalkgeräte, Intubationshohlkörper oder dergl., ausgebildet und mit entsprechend dem weiteren Spülgut (16) zugeordneten Reinigungs- und Trocknungsluftdüsen (17) in vertikaler oder abgewinkelter Form ausgestattet ist, wobei die Düsen (17) gleichermaßen wie die Düsen (5) für die Atemschläuche (4) vom Zuleitungsrohrsystem (7) des Einsatzwagens (1) gespeist sind.

## Claims

1. Method of cleaning and drying respiration tubes (4) in a program-controlled laboratory dishwasher, the respiration tubes (4) being disposed on a supporting frame (2), which is configured as feed carriage (1) and is provided with rinsing water and drying air pipe connections (7, 8, 9) and with associated sets of nozzles (5, DR) over its entire length, ascending in a spiral or helical manner, and the cleaning or rinsing fluid and the drying air being supplied to the ends of the respiration tubes (4) in the respective program steps, characterised in that the respiration tubes (4) are traversed upwardly from below by a cleaning or rinsing liquor column in the "cleaning/rinsing" section of the program and by an air column in the "drying" section of the program.

2. Feed carriage for accomplishing the method according to claim 1, characterised in that the supporting frame (2) for the respiration tubes (4) has one or a plurality of tube depositories (3) in an ascending spiral or helical disposition, in that the one or the plurality of tube depositories (3) terminate at the bottom upstream of separate rinsing water and drying air nozzles (5), which are each combined to form rows of nozzle groups (DR) and to which the respiration tubes (4) are connectable in an untight manner for the emptying of rinsing fluid.

3. Feed carriage according to claim 2, characterised in that at least the lower tube depository (3) of the supporting frame (2) is provided to receive a plurality of respiration tubes (4) in a sequence adjacent and/or above one another, and in that each respiration tube end (4a) has a tube holder (6) associated therewith in the region upstream of the rinsing water and drying air nozzles (5).

4. Feed carriage according to one of claims 2 or 3, characterised in that common nozzles (5) are provided for supplying the cleaning and rinsing fluid and for supplying the drying air to the respiration tubes (4).

5. Feed carriage according to claims 3 and 4, characterised in that the rows of nozzle groups (DR) are disposed behind one another, preferably in a plane upstream of the tube holders (6), and communicate with a common inlet pipe system (7) of the feed carriage (1) having separate conduction paths (8, 9) for the drying air and the rinsing water.

6. Feed carriage according to one or more of claims 2 to 4, characterised in that the tube depositories (3) for the respiration tubes (4) are disposed around a central connection pipe (10) of the supporting frame (2), which pipe is supplied with cleaning or rinsing water from above via one conduction path (8), and the other valve-controlled conduction path for supplying the drying air branches-off from said pipe.

7. Feed carriage according to one or more of claims 2 to 6, characterised in that each nozzle (5), which is associated with a respiration tube (4), is provided with a closure plate (14) for one end (4a) of the respiration tube, said plate being displaceable in the longitudinal direction of the nozzles in opposition to spring pressure (spring 12) and being disposed concentrically on the nozzle shank (13).

8. Feed carriage according to one or more of claims 2 to 7, characterised in that the closure plate (14) is provided with apertures (15) or cutaway portions, in the form of bores, grooves or the like, to achieve the non-watertight lower termination of the respiration tube.

9. Feed carriage according to one or more of claims 2 to 8, characterised in that the remaining space in the feed carriage (1) is configured to accommodate additional articles for rinsing, such as respiration bellows, respiration lime-containing appliances, hollow intubation bodies or the like, and is provided with cleaning and drying air nozzles (17) in vertical or angular form, which nozzles are associated accordingly with the additional articles for rinsing (16), the nozzles (17) being fed by the inlet pipe system (7) of the feed carriage (1) in the same way as the nozzles (5) for the respiration tubes (4).

## Revendications

1. Procédé pour laver et sécher des tubes de respiration (4) dans un lave-vaisselle de laboratoire commandé par un programme, sachant que les tubes de respiration (4) sont posés sur toute leur longueur en forme de spirale ou d'hélice ascendante sur un cadre porteur (2) réalisé en tant que chariot (1) comportant des raccords pour les tuyaux d'eau de rinçage et de l'air de séchage (7, 8, 9) ainsi que des buses (5, DR) qui y sont associées et sachant que le liquide de lavage ou de rinçage ainsi que l'air de séchage alimentent les tubes de respiration (4) par leurs extrémités dans chacune des phases du programme, caractérisé en ce que les tubes de respiration (4) sont parcourus de bas en haut par une colonne d'eau de lavage ou de rinçage au cours de la phase "lavage/rinçage" du programme et par une colonne d'air au cours de la phase "séchage" du programme.

2. Chariot pour mettre en oeuvre le procédé selon la revendication 1, caractérisé en ce que le cadre porteur (2) pour les tubes de respiration (4) possède un ou plusieurs supports de tubes (3) disposés en forme de spirale ou d'hélice ascendante, en ce que le ou les support(s) de tubes (3) se termine(nt) en bas devant des buses d'eau de rinçage ou d'air de séchage (5) séparées qui sont chaque fois regroupées en rangées de groupes de buses (DR) et auxquelles les tubes de respiration (4) peuvent être accouplés de façon non étanche pour les vider du liquide de rinçage.

3. Chariot selon la revendication 2, caractérisé en ce qu'au moins le support de tubes inférieur (3) du cadre porteur (2) est réalisé en une succession d'éléments disposés côte à côte etlou superposés pour supporter plusieurs tubes de respiration (4), et en ce qu'un dispositif de retenue (6) est associé à chaque extrémité de tube de respiration (4a) dans la zone des buses d'eau de rinçage et d'air de séchage (5).

4. Chariot selon l'une des revendications 2 ou 3, caractérisé en ce que des buses (5) communes sont prévues pour amener le liquide de lavage et de rinçage ainsi que l'air de séchage aux tubes de respiration (4).

5. Chariot selon les revendications 3 et 4, caractérisé en ce que les rangées de groupes de buses (DR) sont disposées les unes derrière les autres, de préférence, dans un plan précédant les dispositifs de retenue (6) et sont raccordées à un système de tuyaux d'alimentation (7) commun du chariot (1) avec des conduites séparées (8, 9) pour l'air de séchage et l'eau de rinçage.

6. Chariot selon l'une ou plusieurs des revendications 2 à 4, caractérisé en ce que les supports (3) pour les tubes de respiration (4) sont disposés autour d'une tubulure (10) centrale du cadre porteur (2) qui est alimentée en eau de lavage ou de rinçage par le haut par le biais de l'une des conduites (8), tubulure de laquelle part l'autre conduite commandée par une soupape pour l'alimentation en air de séchage.

7. Chariot selon l'une ou plusieurs des revendications 2 à 6, caractérisé en ce que chaque buse (5) associée à un tube de respiration (4) est munie d'une plaquette de fermeture (14) pour une extrémité de tube de respiration (4a), qui est disposée de manière concentrique sur une tige de buse (13) et qui peut se déplacer dans la direction longitudinale de la buse contre la pression d'un ressort (ressort 12).

8. Chariot selon l'une ou plusieurs des revendications 2 à 7, caractérisé en ce que la plaquette de fermeture (14) est munie d'ouvertures (15) ou d'évidements ayant la forme de perçages, de rainures ou analogues qui forment la fermeture inférieure non étanche du tube de respiration.

9. Chariot selon l'une ou plusieurs des revendications 2 à 8, caractérisé en ce que l'espace inoccupé du chariot (1) est réalisé pour y placer d'autres articles tels que des soufflets d'assistance respiratoire, des cartouches de chaux sodée, des corps creux d'intubation ou analogues, et est équipé de buses d'eau de lavage et d'air de séchage (17) de forme verticale ou courbée qui sont associées aux autres articles à laver (16), sachant que les buses (17) sont alimentées de la même manière que les buses d'eau de rinçage et d'air de séchage (5) pour les tubes de respiration (4) par le système de tuyaux d'alimentation (7) du chariot (1).
